**Europäisches Patentamt**

**European Patent Office**

**Office européen des brevets**

(19)

(11) Veröffentlichungsnummer: **0 582 885 A2**

(12) **EUROPÄISCHE PATENTANMELDUNG**

(21) Anmeldenummer: **93111877.2**

(22) Anmeldetag: **23.07.93**

(51) Int. Cl.5: **G06F 15/20**

(30) Priorität: **05.08.92 DE 4225894**

(43) Veröffentlichungstag der Anmeldung:
**16.02.94 Patentblatt 94/07**

(84) Benannte Vertragsstaaten:
**DE FR GB IT NL**

(71) Anmelder: **Siemens Aktiengesellschaft**
**Wittelsbacherplatz 2**
**D-80333 München(DE)**

(72) Erfinder: **Abraham-Fuchs, Klaus Dipl.-Phys.**
**Graslitzer Strasse 17**
**D-91058 Erlangen(DE)**
Erfinder: **Dr. Schlang, Martin**
**Jäcklinstrasse 5**
**D-81735 München(DE)**
Erfinder: **Uebler, Johann Dipl.-Ing.**
**Effeltricher Strasse 9**
**D-90411 Nürnberg(DE)**

(54) **Verfahren zur Klassifizierung von Feldmustern.**

(57) Bei einem Verfahren zur Klassifizierung von Feldmustern, die von im Innern eines Lebewesens (4) ablaufenden elektrophysiologischen Aktivitäten (8) erzeugt und außerhalb des Lebewesens (4) mit einer Vielkanal-Meßanordnung (12) gemessen werden, ist ein lernfähiger Klassifikator (34) mit der Vielkanal-Meßanordnung (12) verbunden. Der lernfähige Klassifikator (43) weist eine Anzahl von Eingangskanälen (32), denen Merkmalsvektoren zugeführt werden, die repräsentativ für die gemessenen Feldmuster sind. Der lernfähige Klassifikator (34) ist mit Trainingsfeldmustern trainiert, die von einem lokalisierbaren Ersatzmodell der elektrophysiologischen Aktivität (8) erzeugt worden sind. An einem Ausgangskanal (38) wird für jedes Feldmuster ein Wahrscheinlichkeitswert ausgegeben, der die Wahrscheinlichkeit angibt, mit der jedes Feldmuster von dem lokalisierbaren Ersatzmodell erzeugbar ist.

FIG 2

Rank Xerox (UK) Business Services
(3.10/3.09/3.3.4)

EP 0 582 885 A2

Die Erfindung betrifft ein Verfahren zur Klassifizierung von Feldmustern, die von im Innern eines Lebewesens ablaufenden elektrophysiologischen Aktivitäten erzeugt und außerhalb des Lebewesens mit einer Meßanordnung gemessen werden, mit einem lernfähigen Klassifikator, der mit der Meßanordnung verbunden ist.

Ein Verfahren der eingangs genannten Art ist aus der US-PS 5 092 343 bekannt. Dabei wird ein elektromyographisches Signal, das z. B. von einem Muskel über eine Nadel abgegriffen wird, abgetastet und nach typischen Signalformen abgesucht. Die erkannten typischen Signalformen werden dann Mitteln zur Klassifizierung zugeführt, die die typischen Signalformen krankhaften Zuständen zuordnen. Die Mittel zur Klassifizierung umfassen ein neuronales Netzwerk, das mit Daten von normalen und Patientengruppen entsprechend trainiert ist.

In dem Artikel von Pfurtscheller, Flotzinger, Matuschik: "Sleep Classification in Infants Based on Artificial Neural Networks", erschienen in Biomedizinische Technik, Band 37, Heft 6/1992, Seiten 122-130, ist beschrieben, daß zur Schlafklassifikation aus polygraphischen Daten, also Daten mehrerer Vorgänge und Erscheinungen, wie EEG, EOG EMG, EKG, usw., Datenvektoren gebildet werden, die mit Hilfe zweier neuronaler Netzwerke ausgewertet werden. Die neuronalen Netzwerke waren zuvor mit den Klassifikationen eines Experten trainiert worden.

Eine Meßanordnung, mit der räumliche Feldmuster der eingangs genannten Art gemessen werden können, ist aus der EP-A-0 359 864 bekannt. Die Meßanordnung ist als Vielkanal-Meßanordnung ausgebildet und wird auch als biomagnetisches Meßsystem bezeichnet. Damit können die Feldmuster von sehr schwachen Magnetfeldern, die von im Innern eines Lebewesens ablaufenden elektrophysiologischen Aktivitäten erzeugt werden, gemessen werden. Die Vielkanal-Meßanordnung umfaßt eine Vielkanal-Gradiometeranordnung, die mit einer Vielkanal-SQUID-Anordnung gekoppelt ist. Die mit der Vielkanal-Meßanordnung an gleichen Zeitpunkten räumlich getrennt gemessenen Signale, also die Feldmuster, werden herangezogen, ein Ersatzmodell der elektrophysiologischen Aktivität zu bestimmen. Als Ersatzmodell für Aktivitäten sind dort eine Kugel und ein unendlicher Halbraum homogener Leitfähigkeit angegeben, in der sich eine Quelle magnetischer Signale befindet.

Mit dem biomagnetischen Meßsystem können sowohl Magnetoenzephalogramme (MEG) wie auch Magnetokardiogramme (MKG) gemessen werden. Das Hauptziel für der Auswertung der MEG- oder MKG-Aufzeichnungen ist eine dreidimensionale nicht-invasive Lokalisierung von Quellen pathologischer elektrophysiologischen Aktivitäten. Dazu müssen in einem ersten Schritt aus den genannten MEG- oder MKG-Aufzeichnungen die pathologischen Aktivitäten oder Ereignisse bestimmt werden. Pathologische Ereignisse, die im Zerebrum auftreten, sind z.B. Spike-Aktivität, steile Wellen, langsame Wellen, K-Komplexe oder rhythmische Aktivität im Theta- oder Deltabereich.

Die Aufzeichnungsdauer ist in der Größenordnung von 5 bis 10 Minuten, was ca. 30 bis 60 Seiten Aufzeichnungen entspricht. Ein geübter Arzt benötigt zur Bestimmung der pathologischen Ereignisse für eine Seite, die als 30 cm lange Papieraufzeichnung oder als Bild auf einem Computerbildschirm vorliegt, ca. 10 Sekunden. Das bedeutet, daß ungefähr 30 bis 60 "Seiten" nach pathologischen Ereignissen abgesucht werden müssen. Diese Arbeit ist allgemeinen sehr arbeitszeitintensiv und stark ermüdend. Es können auch sehr leicht wichtige Datenabschnitte übersehen werden.

Jedoch können nicht alle ausgewählten pathologischen Aktivitäten oder Ereignisse mit der gleichen Zuverlässigkeit oder Genauigkeit geortet werden. So muß sich z.B. die elektrophysiologische Aktivität durch ein Modell darstellen lassen. Außerdem beeinflußt die Auswahl der Ereignisse, die zur Quellenlokalisation herangezogen werden, wesentlich die Zuverlässigkeit der Lokalisierung, wobei das Signal-Rauschverhältnis entscheidend ist. Werden aufeinander folgende ähnliche Ereignisse gemittelt, ist der Erfolg der Ereigniserkennung von einem gewählten Vergleichsereignis abhängig, womit die zu mittelnden Ereignisse bestimmt werden.

Der Erfindung liegt nun die Aufgabe zugrunde, ein Verfahren anzugeben, mit dem aus den gemessenen Feldmustern Feldmuster bestimmt werden können, die sich mit Hilfe eines Ersatzmodells lokalisieren lassen, wobei den gemessenen Feldmustern Rauschen überlagert sein kann.

Die Aufgabe wird dadurch gelöst, daß die Meßanordnung als Vielkanal-Meßanordnung ausgebildet ist, daß der lernfähige Klassifikator eine Anzahl von Eingangskanälen aufweist, denen Merkmalsvektoren zugeführt werden, die repräsentativ für die gemessenen Feldmuster sind, daß der lernfähige Klassifikator mit Trainingsfeldmustern trainiert ist, die von einem lokalisierbaren Ersatzmodell der elektrophysiologischen Aktivität erzeugt worden sind, und daß an einem Ausgangskanal für jedes Feldmuster ein Wahrscheinlichkeitswert ausgegeben wird, der die Wahrscheinlichkeit angibt, mit der jedes Feldmuster von dem lokalisierbaren Ersatzmodell erzeugbar ist. Im Gegensatz zu vielen bekannten Verfahren, wie z. B. das eingangs genannte Verfahren, die automatisch die von der Meßanordnung gemessenen Meßwerte nach pathologischen Ereignissen absuchen, überläßt die vorliegende Erfindung die Definition der Pathologie der Ereignisse dem Arzt. Erfindungsgemäß werden von der Vorrichtung diejenigen Feldmuster erkannt, die trotz einer mehr oder weniger starken Überlagerung mit Rauschen durch eine vorgegebene Modellquelle oder ein Ersatzmodell lokalisierbar sind. Das Verfahren klassifiziert die Feldmuster in zwei Gruppen:

1. Feldmuster, die mit einer hohen Wahrscheinlichkeit durch das vorgegebene Ersatzmodell erzeugt werden können und die demnach lokalisierbar sind, und

2. Feldmuster, die sich nicht durch das Ersatzmodell beschreiben lassen, weil das Ersatzmodell den zugrunde liegenden physiologischen Prozeß nicht ausreichend oder genau genug beschreibt.

Wegen der vielen möglichen, leicht veränderten Formen der Feldmuster läßt sich die Einteilung in lokalisierbare und nichtlokalisierbare Ereignisse durch ein neuronales Netzwerk besonders sicher lösen.

Eine vorteilhafte Ausgestaltung ist dadurch gekennzeichnet, daß zwischen der Vielkanal-Meßanordnung und dem lernfähigen Klassifikator eine Vorverarbeitungseinheit angeordnet ist, die aus den gemessenen Feldmustern die Merkmalsvektoren erzeugt.

Es ist für die Klassifikation vorteilhaft, die Meßwerte der Feldmuster zu normieren. Damit wird aus den Feldmustern Information entfernt, die für die Klassifikation unbedeutend ist.

Eine weitere vorteilhafte Ausgestaltung zeichnet sich dadurch aus, daß die Vorverarbeitungseinheit eine Amplitudennormierungseinrichtung zur Normierung der Amplituden der in jedem Kanal übertragenen Meßwerte der Feldmuster umfaßt. Damit ist die Klassifizierung der Feldmuster nur abhängig vom gegenseitigen Verhältnis der Meßwerte in den einzelnen Meßwertkanälen.

Eine weitere vorteilhafte Ausgestaltung zeichnet sich dadurch aus, daß der lernfähiges Klassifikator mit einer zweiten Klasse von Trainings-Feldmustern trainiert ist, die von mindestens einem nicht-lokalisierbaren Ersatzmodell erzeugt sind. Das Training des lernfähiges Klassifikators kann über eine Computersimulation erfolgen.

Eine weitere vorteilhafte Ausgestaltung zeichnet sich dadurch aus, daß den Trainings-Feldmustern Rauschen überlagert ist. Durch die Überlagerung von Rauschen ist das neuronale Netzwerk mit vielfach variierten Trainingsfeldmustern trainiert, die den in der Praxis gemessenen Feldmustern entsprechen. Unterschreitet das Signal-Rauschverhältnis in den Feldmustern einen Wert, wird eine Lokalisierung zu ungenau. Dies wird beim Training des neuronalen Netzwerks berücksichtigt.

Eine weitere vorteilhafte Ausgestaltung zeichnet sich dadurch aus, daß die Anzahl der Kanäle in der Vielkanal-Meßanordnung mindestens 30 ist und daß das neuronale Netzwerk versteckte Neuronen aufweist, deren Anzahl ungefähr gleich dem Eineinhalbfachen der Anzahl der Kanäle der Vielkanal-Meßanordnung ist. Die Anzahl der versteckten Neuronen in neuronalem Netzwerk ist ein Kompromiß zwischen der Anzahl der benötigten Trainings-Feldmuster und der umfassenden Fähigkeit des neuronalen Netzwerks, lokalisierbare Feldmuster zu erkennen. Die Anzahl der Eingangsneuronen bestimmt ebenfalls die Anzahl der versteckten Neuronen. Durch die hier gewählte Struktur wird ein Optimum in Bezug auf die Klassifikationsgeschwindigkeit und Genauigkeit der Klassifikation der gemessenen und aufgezeichneten Feldmuster erreicht.

Bei einer weiteren vorteilhaften Ausgestaltung ist das Vielkanal-Meßsystem und der Ausgangskanal des lernfähigen Klassifikators mit einer Bilddarstellungseinheit verbunden, die neben dem zeitlichen Verlauf der Feldmuster auch den Wahrscheinlichkeitswert angibt, mit der das jeweilige Feldmuster durch das Ersatzmodell erzeugbar ist. Damit kann sich der diagnostisierende Arzt bei der Auswertung der Vielkanalaufzeichnungen nur auf die Feldmuster konzentrieren, die durch das verwendete Quellenmodell überhaupt lokalisierbar sind. Andere Feldmuster, die zwar für pathologische Aktivitäten signifikant sind, aber nicht lokalisierbar sind, brauchen in den Aufzeichnungen nicht untersucht zu werden.

Bei einer weiteren vorteilhaften Ausgestaltung ist eine Schwellwerteinrichtung mit dem Ausgangskanal verbunden, der den Wahrscheinlichkeitswert weitergibt, wenn er über einem Schwellwert liegt, und die den Wert Null weitergibt, wenn der Wahrscheinlichkeitswert unter dem Schwellwert liegt. Ist die Wahrscheinlichkeit niedrig, so bedeutet das gleichzeitig, daß eine Lokalisierung der Aktivität mit einem zu großen Fehler behaftet ist, so daß eine Diagnose zu unsicher wird.

Bei einer weiteren vorteilhaften Ausgestaltung umfaßt die Schwellwerteinrichtung eine Zeitstufeneinrichtung, die den Wahrscheinlichkeitswert auf Null setzt, wenn er nicht mindestens für eine Mindestzeitdauer über dem Schwellwert liegt.

Dabei wird von der Erkenntnis ausgegangen, daß elektrophysiologische Aktivitäten eine Mindestdauer aufweisen, die durch das Summen-Aktionspotential vorgegeben ist. Somit ist ein sporadischer kurzzeitiger Anstieg der Wahrscheinlichkeit aus elektrophysiologischen Gründen bei einer pathologischen Aktivität nicht möglich. Eine kurzzeitig erhöhte Wahrscheinlichkeit wird daher unterdrückt und nicht zusammen mit den Feldmustern dargestellt.

Eine weitere vorteilhafte Ausgestaltung zeichnet sich dadurch aus, daß eine Wichtungseinheit mit dem Vielkanal-Meßsystem verbunden ist, die in Abhängigkeit von der Anzahl der positiven und negativen Feldwerte im Feldmuster einen Gewichtsfaktor bildet, und daß eine Multipliziereinheit mit der Wichtungseinheit und dem Ausgangskanal verbunden ist, die den vom Ausgangsneuron ausgegebenen Wahrscheinlichkeitswert mit dem Gewichtsfaktor multipliziert und einen modifizierten Wahrscheinlichkeitswert ausgibt. Die Lokalisierung von elektrophysiologischen Aktivitäten kann am genauesten erfolgen, wenn ein ideales dipolares Feldmuster vorliegt. Bei einem dipolaren Feldmuster mißt die Vielkanal-Meßanordnung mit einer gleiche Anzahl von Meßkanälen eine positive und eine negative Amplitude. Die Lokalisierungsgenauigkeit verschlechtert sich mit einer Zunahme der Anzahl der Meßwerte mit einem gleichen Vorzeichen bis zum schlechtesten Fall eines vollständig monopolaren Feldes. Bei einem monopolaren Feldmuster wird von der Vielkanal-Meßanordnung nur eine Polarität gemessen. Diese Abhängigkeit der Lokalisierungsgenauigkeit wird nicht in die Trainungsphase des lernfähiges Klassifikators eingeführt, jedoch implizit mitgelernt. Die Auswertung der Polarität im Feldmuster kann einfacher von der Wichtungseinheit als von einem entsprechend trainierten Klassifikator vorgenommen werden.

Bei einer weiteren vorteilhaften Ausgestaltung sind die Feldmuster biomagnetische Feldmuster. Die magnetischen Feldmuster lassen sich im Gegensatz zu elektrischen Feldmustern, die ebenfalls von einer elektrophysiologischen Aktivität erzeugt werden, über einfache Ersatzmodelle zur Lokalisierung der Aktivität auswerten. Soll die Lokalisierung aufgrund der Messung der elektrischen Feldmuster erfolgen, muß das Ersatzmodell die unterschiedliche Leitfähigkeit im Innern des Lebewesens berücksichtigen.

Bei einer weiteren vorteilhaften Ausgestaltung besteht das Ersatzmodell aus einem elektrischen Dipol, der in einem Raum angeordnet ist, der eine homogene elektrische Leitfähigkeit aufweist. Mit diesem einfachen Modell lassen sich viele pathologische Aktivitäten diagnostizieren.

Die Erfindung wird im folgenden anhand von drei Figuren beschrieben. Es zeigen:

FIG 1     den Aufbau eines biomagnetischen Meßsystems, mit dem die zu klassifizierenden Feldmuster gemessen werden;

FIG 2     ein Blockdiagramm eines Verfahrens zur Klassifizierung von Feldmustern und

FIG 3     eine Aufzeichnung von Feldmustern, die als von einer Vielkanal-Meßanordnung gemessene Meßwerte vorliegen, sowie die Anzeige der zu den Feldmustern gehörenden Wahrscheinlichkeit.

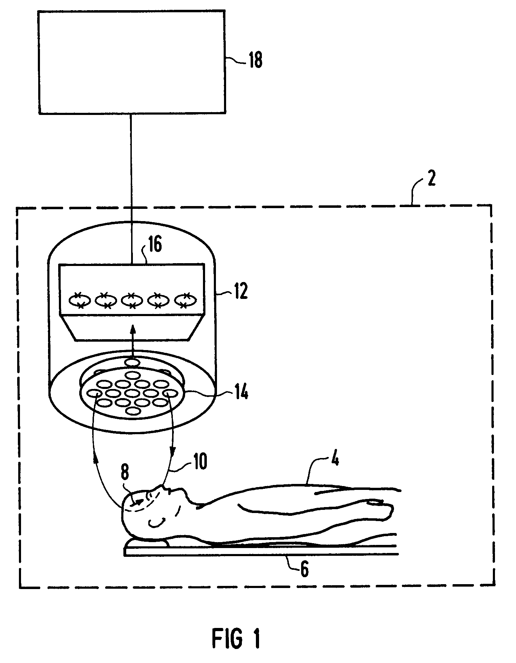

Das Verfahren zur Klassifizierung von Feldmustern wird hier in einem biomagnetischen Meßsystem eingesetzt, mit dem elektrophysiologische Aktivitäten über eine Magnetfeldmessung lokalisiert werden. Dabei können Störungen sowohl außerhalb als auch innerhalb des Patienten selbst erzeugt werden. In FIG 1 ist schematisch eine magnetische Abschirmkammer 2 dargestellt, mit der außerhalb erzeugte Störfelder zum größten Teil abgeschirmt werden. Ein zu untersuchender Patient 4 befindet sich auf einer in der Abschirmkammer 2 angeordneten Patientenliege 6. Elektrophysiologische Aktivitäten, die hier durch einen Pfeil 8 symbolisiert sind, erzeugen ein elektrisches und magnetisches Feldmuster, wobei hier nur das magnetische Feldmuster 10 ausgewertet wird. Dazu wird das Magnetfeld mit einer Vielkanal-Meßanordnung 12 oberhalb des Patienten 4 gemessen. Die Vielkanal-Meßanordnung 12 umfaßt eine Vielkanal-Gradiometeranordnung 14 mit räumlich getrennt angeordneten Gradiometern, die lediglich den Gradienten der Magnetfeldverteilung erfassen und somit schon bei der Messung homogene Störfelder unterdrücken. Hier ist aus Gründen der Übersichtlichkeit eine Vielkanal-Gradiometeranordnung 14 mit 15 einzelnen Gradiometern dargestellt, jedoch werden in der Praxis Vielkanal-Gradiometeranordnungen 14 mit mehr als 30 Kanälen, z.B. 37 Kanäle, verwendet. Die Gradiometer in der Vielkanal-Gradiometeranordnung 14 sind jeweils mit einem SQUID (Super Conducting Quantum Interference Device) verbunden. Die Vielkanal-SQUID-Anordnung 16 und die Vielkanal-Gradiometeranordnung 14 sind in einem Kryostaten angeordnet und dort auf so niedrige Temperatur gehalten, daß Supraleitung vorherrscht.

Die Vielkanal-Meßanordnung 12 ist mittels eines Stativs in einer Untersuchungsposition arretierbar. Die Untersuchungsposition gibt die Meßorte der Gradiometer vor. In der dargestellten Untersuchungsposition werden die Feldmuster von zerebralen Aktivitäten gemessen. Die an den Meßorten zeitlich nacheinander gemessenen Meßsignale werden an eine Signalauswertungseinheit 18 übergeben, die sowohl das zeitliche Verhalten der Meßsignale anzeigt als auch für ausgewählte Feldmuster einen äquivalenten Stromdipol bestimmt, dessen theoretisches Feldmuster dem gemessenen Feldmuster am nächsten kommt. Ein vollständiges Ersatzmodell besteht aus dem Ort des äquivalenten Stromdipols, der Dipolstärke und der Dipolrichtung. Zum Ersatzmodell gehört ebenfalls der Raum, in dem der Stromdipol angeordnet ist. Der Raum, in dem sich der äquivalente Stromdipol befindet, ist im Ersatzmodell für zerebrale Aktivitäten eine Kugel mit homogener Leitfähigkeit und für kardiologischen Aktivitäten ein unendlicher Halbraum mit homogener Leitfähigkeit.

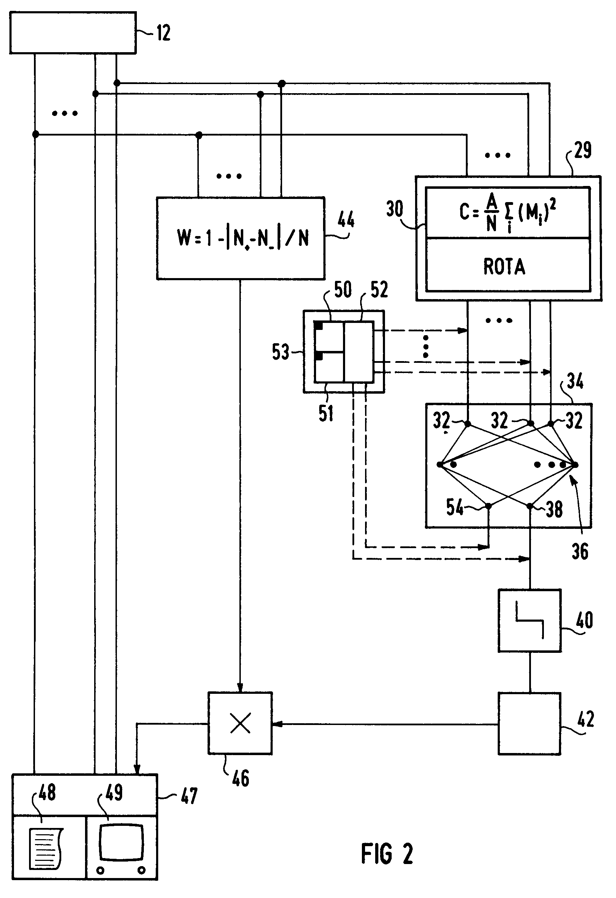

FIG 2 zeigt nun im Blockdiagramm das Verfahren zur Klassifizierung von Feldmustern. Jeweils ein Meßkanal der Vielkanal-Meßanordnung 12 ist über eine Vorverarbeitungseinheit 29, die eine Amplitudennormierungs-Einrichtung 30 umfaßt mit einem Eingangskanal 32 eines lernfähigen Klassifikators Netzwerk 34 verbunden. Die Anzahl der Eingangskanäle 32 entspricht somit der Anzahl der Meßkanäle in der Vielkanal-Meßanordnung 12. Diese Anzahl ist hier 37. Die Vorverarbeitungseinheit 29 normiert die Meßwerte der Feldmuster, um die Klassifikationsgenauigkeit zu erhöhen. So sind z. B. die gemessenen Feldmuster bezüglich einer Rotation um die Symmetrieachse der Vielkanal-Gradiometeranordnung 14 invariant. Deshalb kann das Feldmuster vor der Klassifikation in eine Standardlage gedreht werden, was in FIG 2 durch das Modul ROTA veranschaulicht ist. Damit wird die hier überflüssige Information über den Drehwinkel aus den Feldmustern entfernt. Gegebenenfalls müssen in Abhängigkeit des Drehwinkels und der Geometrie der Vielkanal-Gradiometeranordnung 14 die Meßwerte der Feldmuster interpoliert werden. Ohne eine Interpolation bedeutet die Rotation nichts anders als eine zyklische Vertauschung der Verbindungen zwischen der Vielkanal-Gradiometeranordnung 14 und den Eingangskanälen 32 des Klassifikators 34. Die Reihenfolge der Vertauschung wird aber abhängig vom gemessenen Feldmuster vorgenommen.

Die Amplitudennormierungseinrichtung normiert die Amplituden der Meßwerte, so daß der Summen-Effektivwert über die Amplituden aller Meßwerte eines Feldmusters für alle Feldmuster gleich ist. Dabei wird für jedes Feldmuster ein Faktor A für die Meßwerte des Feldmusters nach folgender Formel bestimmt

$$\frac{A}{N} \sum_{i=1}^{N} (M_i)^2 = C$$

Dabei ist N die Anzahl der Meßkanäle, $M_i$ der Meßwert im i-ten Meßkanal, C eine Konstante, die für alle Feldmuster gilt, und A der gesuchte Faktor.

Als lernfähiger Klassifikator 34 ist hier ein zweischichtiges, vollständig verbundenes, vorwärtsgekoppeltes neuronales Netzwerk mit sigmoidalen Nichtlinearitäten in den Neuronen eingesetzt, wobei die Eingangs- und Ausgangsneuronen des Netzwerks die Eingangs- und Ausgangskanäle des lernfähigen Klassifikators 34 darstellen.

Ein derartiges neuronales Netzwerk ist von J. Hertz, A. Krogh, R. P. Palmer in "Introduction to the Theory of Neural Computation", Adison Wesley, Redwood City, 1991, beschrieben ist. Das neuronale Netzwerk 34 umfaßt 50 versteckte Neuronen 36, wobei die Anzahl der versteckten Neuronen 36 ungefähr gleich dem Eineinhalbfachen der Anzahl der Kanäle der Vielkanal-Meßanordnung 12 ist. Das gibt ein Optimum in der Klassifikationsrate. Hier sind 50 versteckte Neuronen 36 vorgesehen. Ein Ausgangsneuron 38 gibt einen Wahrscheinlichkeitswert aus, der die Wahrscheinlichkeit, mit der das eingangsseitig anliegende Feldmuster mit dem Ersatzmodell eines äquivalenten Stromdipols in einer homogenen, leitfähigen Kugel erzeugbar ist. In der Signalauswertungseinheit 18 wird sowohl die Richtung wie auch der Ort des äquivalenten Stromdipols bestimmt.

Das Ausgangsneuron 38 ist mit einer Schwellwerteinrichtung 40 verbunden, die nur Wahrscheinlichkeitswerte weitergibt, die über einem Schwellwert liegen. Ein Schwellwert von 0,3 ist sinnvoll. Da jedoch ein sporadischer kurzzeitiger Anstieg des Wahrscheinlichkeitswerts aus physiologischen Gründen nicht möglich ist, ist eine Zeitstufeneinrichtung 42 der Schwellwerteinrichtung 40 nachgeordnet, die den Wahrscheinlichkeitswert auf Null setzt, wenn er nicht mindestens für eine Mindestzeitdauer von hier 6 ms über dem Schwellwert liegt.

Eine Wichtungseinheit 44 ist parallel zum neuronalen Netzwerk ebenfalls mit den Meßkanälen der Vielkanal-Meßanordnung 12 verbunden. Die Wichtungseinheit 44 nutzt das empirische Wissen über die Genauigkeit der Lokalisierung von äquivalenten Stromdipolen aus und erzeugt zusätzlich zum Wahrscheinlichkeitswert des neuronalen Netzwerks einen Wichtungswert, der zwischen Null und Eins liegt. Ein äquivalenter Stromdipol kann am genauesten lokalisiert werden, wenn das Feldmusterideal dipolar ist. Ein idealdipolares Feldmuster weist eine gleiche Anzahl von Meßwerten mit einer positiven und einer negativen Amplitude auf. Die Lokalisierungsgenauigkeit nimmt mit abnehmender Dipolarität des Feldmusters ab und ist am schlechtesten bei einem vollständig monopolaren Feld, d.h. alle Meßwerte besitzen dieselbe Amplitudenpolarität. Die Wichtungseinheit 44 erzeugt einen Wichtungswert W nach der Formel

$$W = 1 - |(N_+ - N_- ) / N |,$$

wobei N die Gesamtanzahl der Meßwertkanäle ist, $N_+$ die Anzahl der Meßwerte mit einer positiven und $N_-$ die Anzahl der Meßwerte mit einer negativen Feldamplitude.

Der Ausgang der Wichtungseinheit 44 ist mit einem ersten Eingang einer Multipliziereinheit 46 verbunden, während der Ausgang der Zeitstufeneinrichtung 42 mit einem zweiten Eingang der Multipliziereinheit 46 verbunden ist. Damit wird der vom neuronalen Netzwerk 34 ausgegebene Wahrscheinlichkeitswert mit dem von der Wichtungseinheit 44 ausgegebenen Wichtungswert multipliziert. Die Multipliziereinheit 46 gibt einen modifizierten Wahrscheinlichkeitswert aus.

Der Ausgang der Multipliziereinheit ist zusätzlich zu den Ausgängen der Vielkanal-Meßanordnung mit einer Darstellungs- und Aufzeichnungseinheit 47 verbunden. Die Darstellungs- und Aufzeichnungseinheit 47 umfaßt einen Vielkanalschreiber 48 und einen Bildschirm 49, mit dem die einzelnen Meßwerte und der modifizierte Wahrscheinlichkeitswert aufgezeichnet und/oder angezeigt werden können.

Das neuronale Netzwerk 34 muß zunächst darauf trainiert werden, wie die Feldmuster 10 klassifiziert werden sollen. Seine Leistungsfähigkeit bei der Klassifizierung hängt wesentlich von den verwendeten Trainingsdaten ab. Wegen der vielen möglichen Feldmuster, die durch ein Ersatzmodell erzeugbar sind und die ggf. noch mit Rauschen überlagert sind, ist zum Trainieren des neuronalen Netzwerks 34 eine große Menge von Trainingsdaten erforderlich, die in einer Computersimulation erzeugt werden können. Eine erste Klasse von Trainings-Feldmustern, die als "richtige Klasse" bezeichnet ist, enthält Feldmuster, die von einen lokalisierbaren Ersatzmodell erzeugt sind. Eine zweite Klasse Trainings-Feldmustern, die als "falsche Klasse" bezeichnet ist, enthält nicht-lokalisierbare Feldmuster.

Zum Erzeugen der Trainingsdaten für die "richtige Klasse" wird von einem äquivalenten Stromdipol ausgegangen, der in einer Kugel mit einer homogenen Leitfähigkeit angeordnet ist. Lokalisierbar sind Stromdipole, die sich in der gesamten Hälfte der Kugel, die der Vielkanal-Meßanordnung 12 zugewandt ist, befinden und Stromdipole, die ein kleines Stück unterhalb dieser Halbkugel angeordnet sind. Der Kugelradius beträgt typisch 9 cm. Ausgeschlossen ist eine kleinere zentrale Kugel mit einem Radius von 2 cm, weil Stromdipole, die innerhalb dieser kleinen Kugel liegen, nur sehr kleine Magnetfelder außerhalb der Modellkugel erzeugen. Dieses, im wesentlichen halbkugelschalenförmige Gebiet wird im folgenden als "Region of Interest" oder ROI bezeichnet.

Eine simulierte Vielkanal-Meßanordnung, die der Vielkanal-Meßanordnung 12 entspricht, ist 12 cm oberhalb der ROI angeordnet. Die Trainings-Feldmuster für die "richtige Klasse" werden durch statistisch verteilte Stromdipole innerhalb dieser ROI erzeugt. Dabei werden sowohl die Lokalisierungsals auch Orientierungsparameter des äquivalenten Stromdipols statistisch variiert. Die Feldmuster sind normiert auf einen Effektivwert von 1 pT.

Damit sind die Feldmuster unabhängig von der Amplitude und die Amplitude geht nicht ein in die Klassifizierung durch das neuronale Netzwerk 34. Allgemein sind die Trainings-Feldmuster ebenso vorverarbeitet, wie es für die gemessenen Feldmuster durch die Vorverarbeitungseinheit 29 geschieht. Diese Klasse von Trainingsdaten wird im folgenden als Klasse I bezeichnet.

Zur Erzeugung der Trainings-Feldmuster für die "falsche Klasse" muß berücksichtigt werden, welche Arten von Feldmustern überhaupt auftreten und gemessen werden können. Neben Feldmustern, die von einer fokalen Aktivität herrühren und die durch das Ersatzmodell erzeugbar sind, wird ein großer Teil der Feldmuster durch multifokale Aktivitäten erzeugt, die über das gesamte Untersuchungsgebiet verteilt sind. Es wird hier angenommen, daß die Anzahl Stromdipole, die an der multifokalen Aktivitäten beteiligt sind, begrenzt ist, so daß die Feldmuster trotzdem eine systematische Struktur aufweisen. Zur Erzeugung der entsprechenden Trainings-Feldmuster werden hier gleichzeitig drei Stromdipole variiert, die statistisch im ROI verteilt sind. Im folgenden werden die Feldmuster der statistisch verteilten drei Stromdipole in eine Klasse II eingeordnet. Alle anderen komplexeren Aktivitäten, einschließlich der Perioden von sehr niedrigen Aktivitäten, wo Rauschen dominiert, werden simuliert durch Zufallsrauschmuster über alle Meßkanäle: Das Rauschen ergibt die Klasse III. Auch die Amplituden der Feldmuster der Klassen II und III werden auf einen Effektivwert von 1 pT normiert.

Die Computersimulation erzeugt zunächst eine statistische Mischung der Feldmuster aller drei Klassen und speichert die Feldmuster und die dazugehörige Klasse. In den Fällen, wo die Feldmuster von einem oder drei äquivalenten Stromdipolen erzeugt werden, werden die Parameter der äquivalenten Stromdipole ebenfalls gespeichert.

Die Meßwerte der Feldmuster, die von äquivalenten Stromdipolen nachgebildet werden können, treten in der Praxis mit sehr verschiedenen Signal-Rauschverhältnissen auf. Da die Fähigkeit des neuronalen Netzwerks 34 zur Klassifizierung auch vom Rauschpegel abhängt, müssen die Trainings-Feldmuster verschiedene Rauschpegel enthalten. Dazu werden sowohl rauschfreie Muster als auch Muster mit einem relativ niedrigen Signal-Rauschverhältnis von 3 erzeugt. Das SignalRauschverhältnis für die verrauschten Trainings-Feldmuster ist definiert als das Verhältnis des Effektivwertes der unverrauschten Feldmuster dividiert durch den Effektivwert des Rauschens, das hinzugefügt ist.

Bevor nun diese Feldmuster zum Trainung des neuronalen Netzwerks 34 benutzt werden ist eine Neuklassifikation der Feldmuster erforderlich. Die genaue Zielsetzung für die Klassifizierung von Feldmustern lautet: Es sollen Feldmuster erkannt werden, welche mit großer Wahrscheinlichkeit von einer fokalen Aktivität stammen, die durch einen äquivalenten Stromdipol beschrieben und lokalisiert werden kann. Nicht alle Feldmuster der Klasse I erfüllen dieses Kriterium, weil einige der statistisch verteilten äquivalenten Stromdipole in der Nähe des Mittelpunktes liegen können und somit kein nennenswertes magnetisches Feld erzeugen. Andererseits kann in der multifokalen Klasse II einer der drei äquivalenten Stromdipole über die anderen beiden dominieren, weil er deutlich näher an der Vielkanalmeßanordnung 12 angeordnet ist als die anderen beiden. Es können sich auch zwei von den drei Stromdipolen gegenseitig aufheben. Das neuronale Netzwerk 34 würde somit fehlgeleitet, solche Muster als multifokale Feldmuster zu lernen. Aus diesem Grund ist es notwendig, die Trainings-Feldmuster der Klasse I und II vorher einem Stromdipol-Lokalisierungsverfahren zu unterwerfen und das Ergebnis der Lokalisierung mit den wahren oder gespeicherten Parametern des äquivalenten Stromdipols zu vergleichen. Eine willkürlich gewählte Grenze von z.B. 1,5 cm Lokalisierungsgenauigkeit, d.h. die Differenz zwischen dem Lokalisierungsergebnis und dem wahren Dipolort, wird daher zwischengeschaltet. Alle Muster der Klassen I und II, die innerhalb der oben angegebenen Grenze erfolgreich lokalisiert werden können, werden danach in die erste "richtige Klasse" klassifiziert. Alle Feldmuster der ursprünglichen Klasse I, die nicht erfolgreich lokalisiert werden können werden in die zweite "falsche Klasse" eingeordnet. Alle nicht lokalisierten Feldmuster der Klasse II kommen wie die Feldmuster der Klasse III zur zweiten Klasse. Diese so erzeugten Trainingsfeldmuster der ersten und zweiten Klasse werden benutzt, um das neuronale Netzwerk 34 zu trainieren. In FIG 2 sind die Trainings-Feldmuster der ersten Klasse in einem ersten Speicher 50 und die Trainings-Feldmuster der zweiten Klasse sind in einem zweiten Speicher 51 abgelegt.

Die Trainingsdaten werden benutzt, um die internen Verbindungen, die sogenannten Gewichte des Netzwerks 34, zu bestimmen. Zu Beginn des Trainings sind alle Gewichte mit zufälligen Werten belegt. Zum Training des Netzwerks 34 wird nun ein Feldmuster aus der ersten oder zweiten Klasse von einer Auswahleinheit 52 mit einem Zufallsgenerator aus dem Speicher 50 oder 51 abgerufen und den Eingangsneuronen 32 zugeführt. In FIG 2 ist die Trainingsphase durch die gestrichelten Verbindungen mit einem Trainingsmodul 53, das die Speicher 50, 51 und die Anwahleinheit 52 umfaßt, veranschaulicht.

Zum Training des neuronalen Netzwerks 34 wird neben dem Ausgangsneuron 38 noch ein weiteres Ausgangsneuron 54 benötigt. Das Ausgangsneuron 38 gibt einen Wahrscheinlichkeitswert für die Wahrscheinlichkeit aus, daß das Feldmuster von einem äquivalenten Stromdipol erzeugbar ist. Dagegen gibt das Ausgangsneuron 54 gibt einen Wahrscheinlichkeitswert dafür aus, daß das Feldmuster nicht von einem äuqivalenten Stromdipol erzeugbar ist. Die Summe der beiden Wahrscheinlichkeiten ist nicht zwangsläufig "1", weil es sich um ein nichtlineares Netzwerk 34 handelt. Bei der Klassifizierung der Feldmuster hat dasjenige Ausgangsneuron 38 oder 54 "gewonnen", dessen Wert am größten ist. Erzeugt z.B. ein Feldmuster am Ausgangsneuron 38 den Wert "0,6" und am Ausgangsneuron 54 den Wert "0,55", so ist das Feldmuster als lokalisierbar erkannt.

Diese Einteilung wird verglichen mit der wahren Klassifizierung des Feldmusters. Mit Hilfe der Fehlerrückverfolgung entsprechend wie von D.E. Rummelhart, J.L. McClelland in dem Artikel "Parallel distributed processing", MIT Press, 1986 angegeben ist, werden die Gewichte im Netzwerk 34 so angepaßt, daß die Differenz zwischen dem Ausgangswert im Mittel minimiert ist über alle Trainings-Feldmuster. Der Trainingsprozeß wird solange wiederholt bis die richtige Klassifikationsrate des Netzwerks 34 maximal ist. Es hat sich als ausreichend herausgestellt, daß mit 10 000 Trainings-Feldmustern eine optimale Klassifikationsrate nach $1,5 \times 10^7$-malige Training der einzelnen Muster erreicht wurde. Das Training selbst benötigt mehrere Stunden Rechnerzeit auf einer RISK-Work-Station. Es muß jedoch nur einmal durchgeführt werden. Im Gegensatz dazu ist der Klassifizierungsprozeß der gemessenen Feldmuster sehr schnell. Er liegt in der Größenordnung von einigen Minuten für in einem Zeitbereich von einer Minute gemessene Feldmuster, die mit einer Abtastrate von 400 Hz aufgezeichnet wurden.

Obwohl das Training nur für eine einzige simulierte Position der Vielkanal-Meßanordnung 12 in Bezug zur ROI durchgeführt wurde, ist wegen der Kugelsymmetrie mit ähnlichen Bedingungen bei den meisten Magnetenzephalogramm zu rechnen. Abweichungen können sich ergeben durch einen vergrößerten Abstand der Vielkanal-Meßanordnung 12 oder eine seitliche Verschiebung der Vielkanal-Meßanordnung 12 in Bezug auf den Mittelpunkt des ROI. Berücksichtigt man jedoch die Größe des menschlichen Kopfes und die Größe der Fläche der Vielkanal-Meßanodnung 12, dann betragen beide Abweichungen nicht mehr als wenige Zentimeter. Derartig kleine Abweichungen haben keinen wesentlichen Einfluß auf die Trainins-Feldmuster.

Wenn die Anordnung der Gradiometer in der Vielkanal-Gradiometeranordnung 14 geändert wird, muß das neuronale Netzwerk 34 im Prinzip auch neu trainiert werden. Kleine Änderungen in der Anordnung können jedoch durch Interpolation der Feldmuster ausgeglichen werden.

Generell kann die Vorrichtung zur Klassifikation von Feldmustern auch elektrische Feldmuster aus Elektroenzephalogrammen oder Elektrokardiogrammen klassifizieren. Jedoch muß dann das Ersatzmodell die unterschiedliche Leitfähigkeit des Körpers berücksichtigen, weil durch Leitfähigkeitsänderungen im ROI Feldverzerrungen auftreten, die die Lokalisierungsgenauigkeit beeinträchtigt.

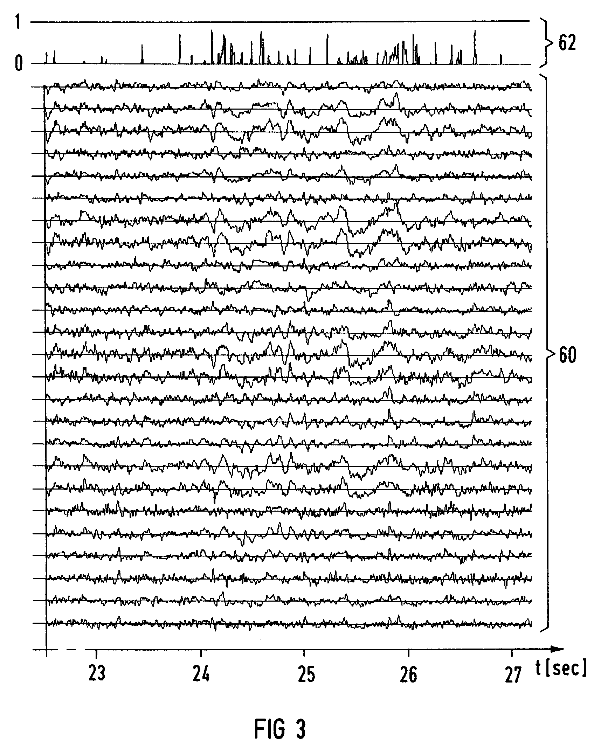

FIG 3 zeigt nun eine Aufzeichnung von mit einer Vielkanal-Meßanordnung 12 gemessenen Feldmuster. Ein Feldmuster ist gegeben durch zum gleichen Zeitpunkt gemessenen Meßwerte 60. Oberhalb der Meßwerte 60 ist in einem Diagramm 62 der Wahrscheinlichkeitswert aufgetragen, der zu jedem Feldmuster gehört und der die Wahrscheinlichkeit angibt, mit der das Feldmuster von dem gewählten Ersatzmodell erzeugbar ist. Der Wahrscheinlichkeitswert liegt zwischen Null und Eins.

**Patentansprüche**

1.	Verfahren zur Klassifizierung von räumlichen Feldmustern, die von im Innern eines Lebewesens (4) ablaufenden elektrophysiologischen Aktivitäten (8) erzeugt und außerhalb des Lebewesens mit einer Meßanordnung gemessen werden, mit einem lernfähigen Klassifikator (34), der mit der Meßanordnung verbunden ist, **dadurch gekennzeichnet,** daß die Meßanordnung als Vielkanal-Meßanordnung (12) ausgebildet ist, daß der lernfähige Klassifikator (34) eine Anzahl von Eingangskanälen (32) aufweist, denen Merkmalsvektoren zugeführt werden, die repräsentativ für die gemessenen Feldmuster sind, daß der lernfähige Klassifikator (34) mit Trainingsfeldmustern trainiert ist, die von einem lokalisierbaren Ersatzmodell der elektrophysiologischen Aktivität (8) erzeugt worden sind, und daß an einem Ausgangskanal (38) für jedes Feldmuster ein Wahrscheinlichkeitswert ausgegeben wird, der die Wahrscheinlichkeit angibt, mit der jedes Feldmuster von dem lokalisierbaren Ersatzmodell erzeugbar ist.

2.	Verfahren nach Anspruch 1, **dadurch gekennzeichnet,** daß zwischen der Vielkanal-Meßanordnung (12) und dem lernfähigen Klassifikator (34) eine Vorverarbeitungseinheit (29) angeordnet ist, die aus den gemessenen Feldmustern die Merkmalsvektoren erzeugt.

3.	Verfahren nach Anspruch 2, **dadurch gekennzeichnet,** daß die Vorverarbeitungseinrichtungseinrichtung (29) eine Amplitudennormierungs-Einrichtung (30) zur Normierung der Amplituden der in jedem Kanal übertragenen Meßwerte umfaßt.

4.	Verfahren nach einem der Ansprüche 1 bis 3, **dadurch gekennzeichnet, daß** der lernfähige Klassifikator (34) mit einer zweiten Klasse von Trainings-Feldmustern trainiert ist, die von mindestens einem nichtlokalisierbaren Ersatzmodell erzeugt sind.

5.	Verfahren nach Anspruch 4, **dadurch gekennzeichnet,** daß den Trainings-Feldmustern Rauschen überlagert ist.

6.	Verfahren nach einem der Ansprüche 1 bis 7, **dadurch gekennzeichnet,** daß ein mindestens zweischichtiges neuronales Netzwerk (34) mit Eingangsneuronen (32) und Ausgangsneuronen (38) als lernfähiger Klassifikator (34) eingesetzt wird, wobei die Eingangs- und Ausgangsneuronen (32,38) die Eingangs- und Ausgangskanäle (32,38) darstellen.

7.	Verfahren nach Anspruch 6, **dadurch gekennzeichnet,** daß die Anzahl der Eingangsneuronen (32) gleich ist der Anzahl der Kanäle der Vielkanal-Meßanordnung (12).

8.	Verfahren nach Ansprüche 5 oder 6, **dadurch gekennzeichnet,** daß die Anzahl der Kanäle in der Vielkanal-Meßanordnung (12) mindestens 30 ist und daß das neuronale Netzwerk (34) versteckte Neuronen (36) aufweist, deren Anzahl ungefähr gleich dem Eineinhalbfachen der Anzahl der Kanäle der Vielkanal-Meßanordnung (12) ist.

9.	Verfahren nach einem der Ansprüche 1 bis 8, **dadurch gekennzeichnet,** daß das Vielkanal-Meßsystem (12) und der Ausgangskanal (38) des lernfähigen Klassifikators (32) mit einer Bilddarstellungseinheit (47,48,49) verbunden ist, die neben dem zeitlichen Verlauf der Feldmuster auch den Wahrscheinlichkeitswert anzeigt, mit der das jeweilige Feldmuster durch das Ersatzmodell erzeugbar ist.

10.	Verfahren nach einem der Ansprüche 1 bis 9, **dadurch gekennzeichnet,** daß eine Schwellwerteinrichtung (40) mit dem Ausgangskanal (38) verbunden ist, die den Wahrscheinlichkeitswert weitergibt, wenn er über einem Schwellwert liegt, und die den Wert Null weitergibt, wenn der Wahrscheinlichkeitswert unter dem Schwellwert liegt.

11.	Verfahren nach Anspruch 10, **dadurch gekennzeichnet,** daß der Schwellwerteinrichtung (40) eine Zeitstufeneinrichtung (42) nachgeordnet ist, die den Wahrscheinlichkeitswert auf Null setzt, wenn er nicht mindestens für eine Mindestzeitdauer über dem Schwellwert liegt.

12.	Verfahren nach einem der Ansprüche 1 bis 11, **dadurch gekennzeichnet,** daß eine Wichtungseinheit (44) mit dem Vielkanal-Meßsystem (12) verbunden ist, die in Abhängigkeit von der Anzahl der positiven und negativen Feldwerte des Feldmusters einen Gewichtsfaktor bildet, und daß eine Multipliziereinheit (46) mit der Wichtungseinheit (44) und dem Ausgangskanal (38) verbunden ist, die den vom Ausgangskanal ausgegebene Wahrscheinlichkeitswert mit dem Gewichtsfaktor multipliziert und einen modifizierten Wahrscheinlicheitswert ausgibt.

13. Verfahren nach einem der Ansprüche 1 bis 12, **dadurch gekennzeichnet,** daß die Feldmuster biomagnetische Feldmuster sind.

14. Verfahren nach einem der Ansprüche 1 bis 13, **dadurch gekennzeichnet,** daß das Ersatzmodell aus einem elektrischen Stromdipol besteht, der in einem Raum angeordnet ist, der eine homogene elektrische Leitfähigkeit aufweist.

15. Verfahren nach Anspruch 14, **dadurch gekennzeichnet,** daß der Raum eine leitfähige Kugel ist.

FIG 1

$$W = 1 - \left| N_+ - N_- \right| / N$$

$$C = \frac{A}{N} \sum_i (M_i)^2$$

ROTA

FIG 2

FIG 3